# EUROPEAN PATENT SPECIFICATION

(11) **EP 3 181 039 B1**
(45) Date of publication and mention of the grant of the patent: **11.09.2019**
(21) Application number: 15828305.1
(22) Date of filing: 27.07.2015
(51) Int. Cl.: A61B 5/0245, A61B 5/022, A61B 5/024, A61B 5/026

(54) **BIOLOGICAL INFORMATION READING DEVICE**
VORRICHTUNG ZUM LESEN BIOLOGISCHER INFORMATIONEN
DISPOSITIF DE LECTURE D'INFORMATIONS BIOLOGIQUES

(30) Priority: 28.07.2014 JP 2014153171
(43) Date of publication of application: 21.06.2017
(73) Proprietor: Shinano Kenshi Co., Ltd., Ueda-shi, Nagano 386-0498 (JP)
(72) Inventor: NAKAMURA, Hiroyuki, Ueda-shi, Nagano 386-0498 (JP)
(74) Representative: Schäfer, Matthias W.
(86) International application number: PCT/JP2015/071244
(87) International publication number: WO 2016/017579

(56) References cited:
- WO-A1-2006/097910
- WO-A1-2013/023071
- JP-A- H05 207 978
- JP-A- H07 275 226
- JP-A- 2005 066 087
- JP-A- 2012 254 194
- HOI LAM TAM ET AL: "Integration of transmissible organic electronic devices for sensor application", PROCEEDINGS OPTICAL DIAGNOSTICS OF LIVING CELLS II, vol. 8831, 18 September 2013 (2013-09-18), page 883121, XP055424467, US ISSN: 0277-786X, DOI: 10.1117/12.2025493 ISBN: 978-1-5106-1354-6
- G E C ELLERBY ET AL: "Validation of a spectroscopic sensor for the continuous, noninvasive measurement of muscle oxygen saturation and pH", PHYSIOLOGICAL MEASUREMENT., vol. 34, no. 8, 17 July 2013 (2013-07-17), pages 859-871, XP055425110, GB ISSN: 0967-3334, DOI: 10.1088/0967-3334/34/8/859
- Zuckerman D. Bram: "Summary of Safety and Effectiveness for CareGuide Oximeter", Reflectance Medical, Inc. - 510(k) Premarket Notification Submission: CareGuide' M Oximeter, 26 July 2012 (2012-07-26), pages 1-4, XP055425298, Retrieved from the Internet: URL:https://www.accessdata.fda.gov/cdrh_do cs/pdf11/K113656.pdf [retrieved on 2017-11-15]
- MARC KOETSE ET AL: "In plane optical sensor based on organic electronic devices", PROCEEDINGS OPTICAL DIAGNOSTICS OF LIVING CELLS II, vol. 7054, 25 August 2008 (2008-08-25), page 70541I, XP055423555, US ISSN: 0277-786X, DOI: 10.1117/12.794830 ISBN: 978-1-5106-1354-6
- JOSEPH SHINAR ET AL: "Organic light-emitting devices (OLEDs) and OLED-based chemical and biological sensors: an overview", JOURNAL OF PHYSICS D: APPLIED PHYSICS, vol. 41, no. 13, 12 June 2008 (2008-06-12) , page 133001, XP055507413, GB ISSN: 0022-3727, DOI: 10.1088/0022-3727/41/13/133001

## Description

### {Technical Field}

The present invention relates to a biological information reading device.

### {Background Art}

Various biological information reading technologies are present. When a blood pressure is used as an example of biological information, a device for estimating a blood pressure by measuring a pulse wave of a measured person is disclosed, for example, in PTL 1 and PTL 2. Further, NPL 1 discloses a method of estimating a blood pressure taking arteriosclerosis into consideration from a pulse wave propagation time. The blood pressure of the measured person can be measured using the device or the method.

### {Citation List}

### {Patent Literature}

{PTL 1} JP 2011-200262 A
{PTL 2} JP 1-214339 A

### {Non Patent Literature}

{NPL 1} IEEJ Trans. EIS, Vol.130, No.2, 2010, "2010 The Institute of Electrical Engineers of Japan", "Cuffless Blood Pressure Estimation with Photoplethysmograph Signal by Classifying on Account of Cardiovascular Characteristics of Old Aged Patients", Satomi Suzuki, Koji Oguri

### {Summary of Invention}

### {Technical Problem}

PTL 1 proposes a vascular pulse wave measurement system, which measures a vascular pulse wave using light, as a biological information reading device. The vascular pulse wave measurement system of PTL 1 irradiates a skin of a living body with light using a light emitting diode (LED) as a light emitting element, receives light diffused and reflected on the skin using a light receiving element, and outputs a pulsation waveform as a time change of a frequency from the received light.

It is important to continuously read biological information and examine a change thereof in an object of perceiving an indication of a serious disease. For example, when a daily variation in blood pressure of a measured person can be measured, it is possible to discover a maximum value, a minimum value, or a rapid change in a short time of a maximum/minimum blood pressure in a daily life. To this end, it is desired to regularly acquire a blood pressure of the measured person. However, the device disclosed in PTL 1 and PTL 2 is an extension of/substitution for an existing sphygmomanometer intended for improvement in convenience of measurement, and furthermore, the device is large and disturbs a free action of the measured person. In addition, in the device of PTL 1, a light sensor circuitry to be attached to a skin of the measured person lacks flexibility, and thus cannot absorb an individual difference in body shape of a measured person, and there is a need for installation methods which continuously apply a strong pressure at all times. As a result, the device has been unsuitable to be used by being attached to the skin at all times in terms of installation stress or pressure necrosis.

Therefore, it is difficult to acquire a blood pressure value of the measured person at all times using the device of PTL 1 and PTL 2 or the like, and as a result, perception of an indication of outbreak/return of the serious disease of the measured person cannot be expected. The above description is not restricted to the blood pressure, and is similarly applied to a case in which other biological information is read.

In addition, difficulty is entailed when the measured person makes a determination with regard to a result of continuously reading biological information. For example, even when continuous biological information shows an indication of a brain disease, the measured person needs knowledge for reading and understanding a change in biological information in order to recognize the indication. However, in general, this is a territory for an expert. Thus, a valuable indication is not utilized for an advance quick reaction of the measured person, and the measured person is unfortunately in a serious state in some cases.

Further, not a few indications of serious diseases develop in a short time. Even when the biological information can be continuously read, the measured person needs to be conscious of a tendency of the biological information at all times, which is remarkably annoying in living a fulfilling daily life.

In addition, an LED is a point light source, and thus heat generation is concentrated on one point to cause low temperature burn injury on a skin in some cases, and in a blood oxygen concentration measurement device (a pulse oximeter) using an LED as a light emitting element, there is a case in which a skin of a child is thermally burned. Thus, in a case in which an LED is used as the light emitting element, a problem of heat generation is desired to be solved to continuously measure biological information for a long time. Further, the disclosure of the following documents belongs to the prior art: WO 2006/(097910 A1; Hoi Lam Tam et al., Integration of transmissible organic electronic devices for sensor application, Proceedings Optical Diagnostics of Living Cells II, 18 September, 2013; and Joseph Shinar et al., Organic Light-Emitting Devices (OLEDs) and OLED-Based Chemical and Biological Sensors: An Overview, Journal of Physics D: Applied Physics, 12 June 2008.

Therefore, there has been a strong desire for a portable device capable of continuously acquiring biological information at all times by being flexibly attached to a skin of a measured person without the measured person feeling inconvenience, analyzing the acquired biological information without delay, and issuing an alert to the measured person in the case of danger.

The present invention has been conceived under the above-described background, and an object of the present invention is to provide a biological information reading device capable of continuously acquiring biological information such as a blood pressure of a measured person or the like at all times.

### {Solution to Problem}

The above technical problem has been solved by a biological infomration reading device having the features of claim 1. Advantageous embodiments are described in the dependent claims. An aspect of the present invention is a biological information reading device for reading biological information, comprising: a biological signal acquisition unit for acquiring a biological signal from a living body; an operation unit for performing an operation of estimating biological information based on an acquired biological signal corresponding to the biological signal acquired by the biological signal acquisition unit; and a biological information output unit for outputting estimated biological information corresponding to the biological information estimated by the operation unit to an outside of the biological information reading device.

Further, in the present invention, the biological signal acquisition unit may include a stacked organic light emitting and receiving element. In addition, it is preferable to provide a light transmissive adhesive layer on a surface coming into contact with a skin of the living body.

Further, the biological information reading device of the present invention may include an estimated biological information time series storage unit that successively stores the estimated biological information subjected to the operation of the operation unit over time.

Further, the biological information reading device of the present invention may include a determination unit that determines a state of the living body based on the estimated biological information; and a determination information holding unit that holds information necessary for the determination unit to perform a determination.

Further, the biological information reading device of the present invention may include a message output unit that outputs a message according to a determination result of the determination unit.

### {Advantageous Effects of Invention}

According to the present invention, it is possible to continuously acquire biological information such as a blood pressure of a measured person or the like at all times. In addition, it is possible to identify an indication of a disease at all times based on the biological information acquired at all times.

### {Brief Description of Drawings}

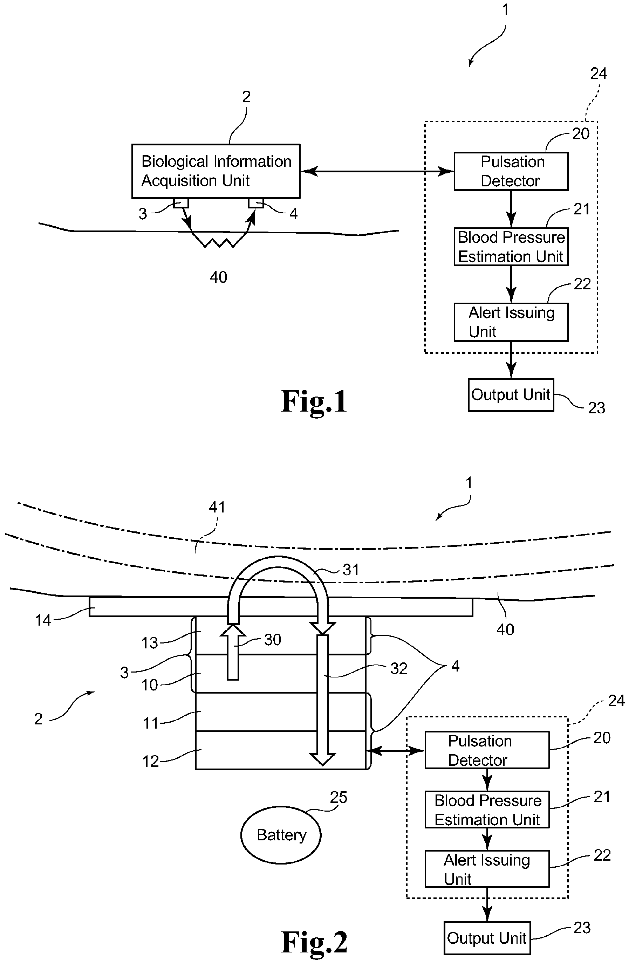

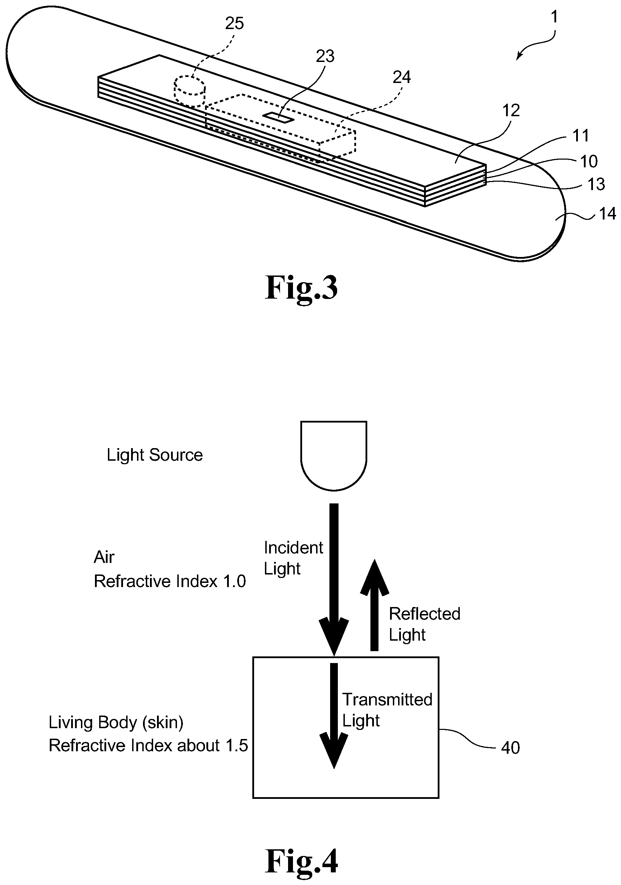

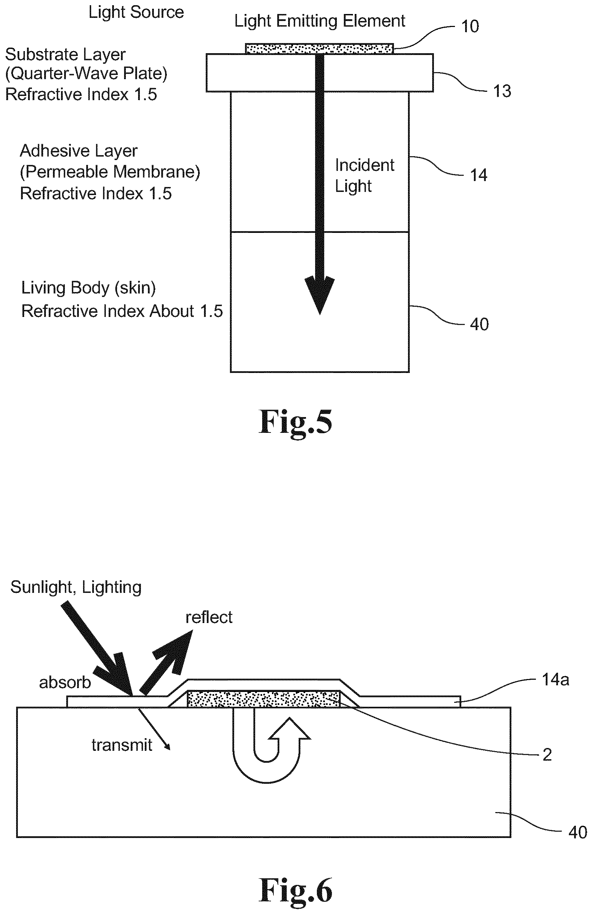

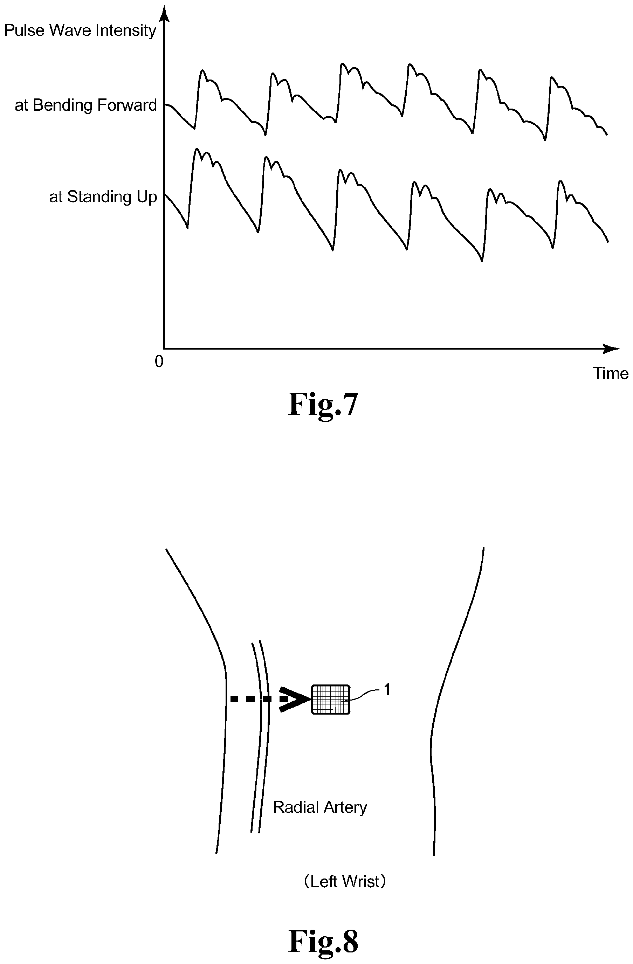

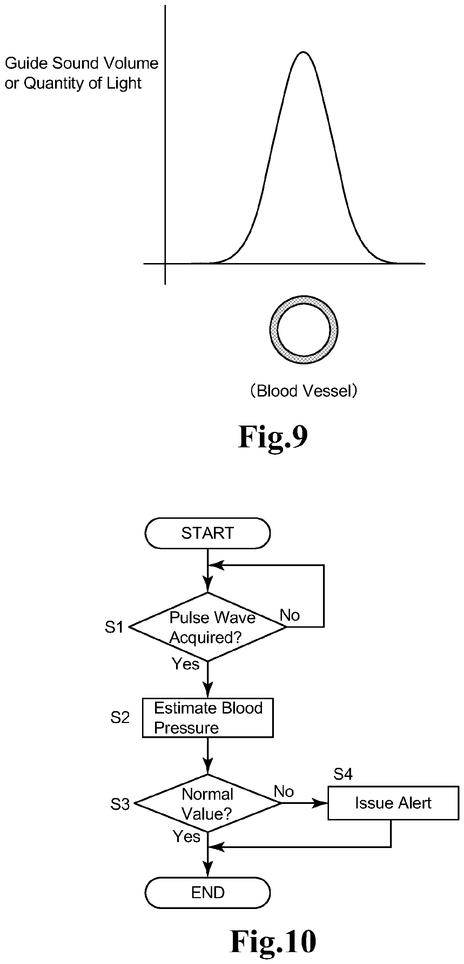

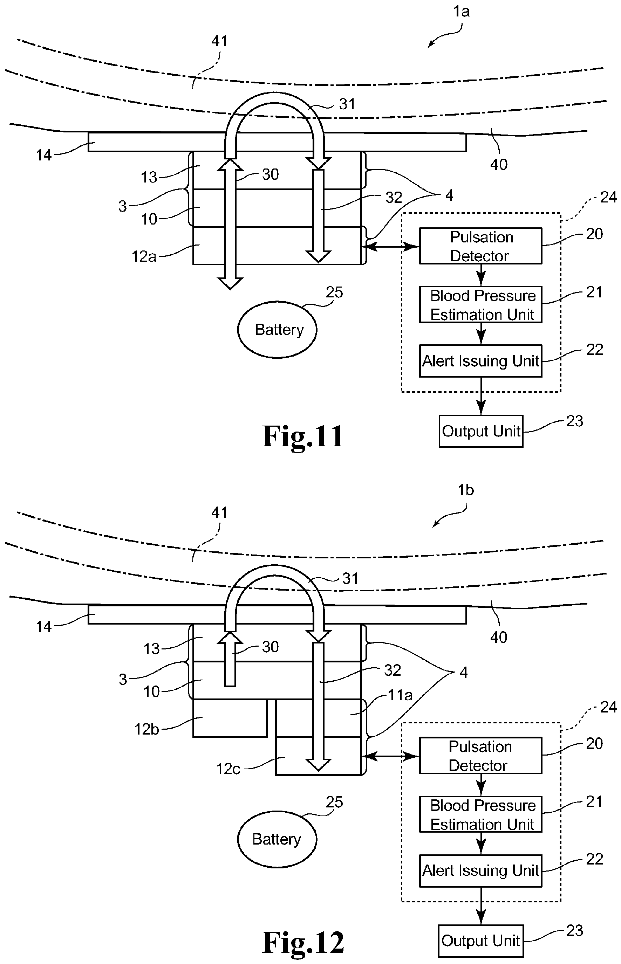

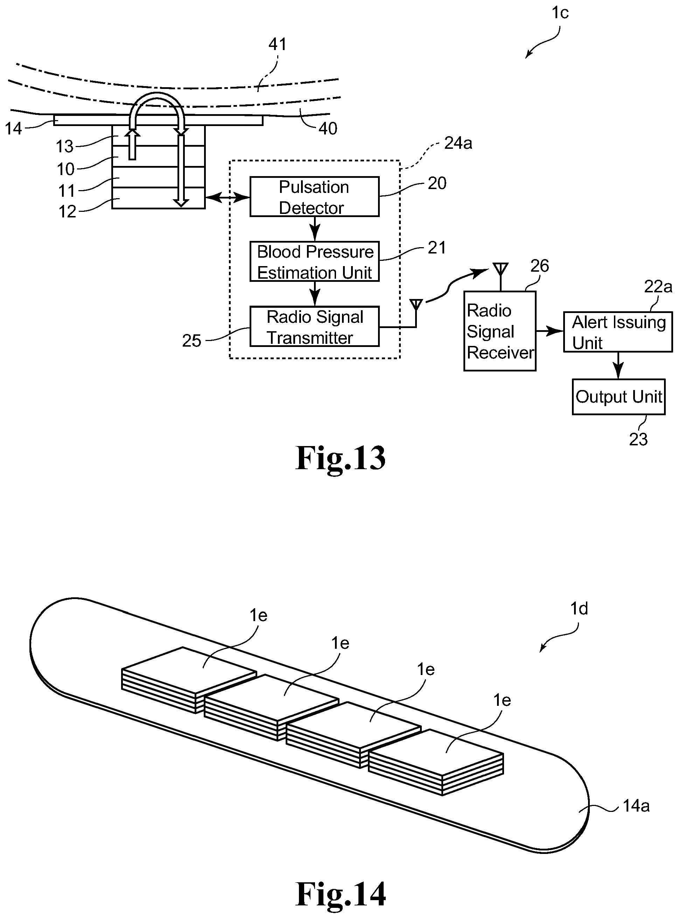

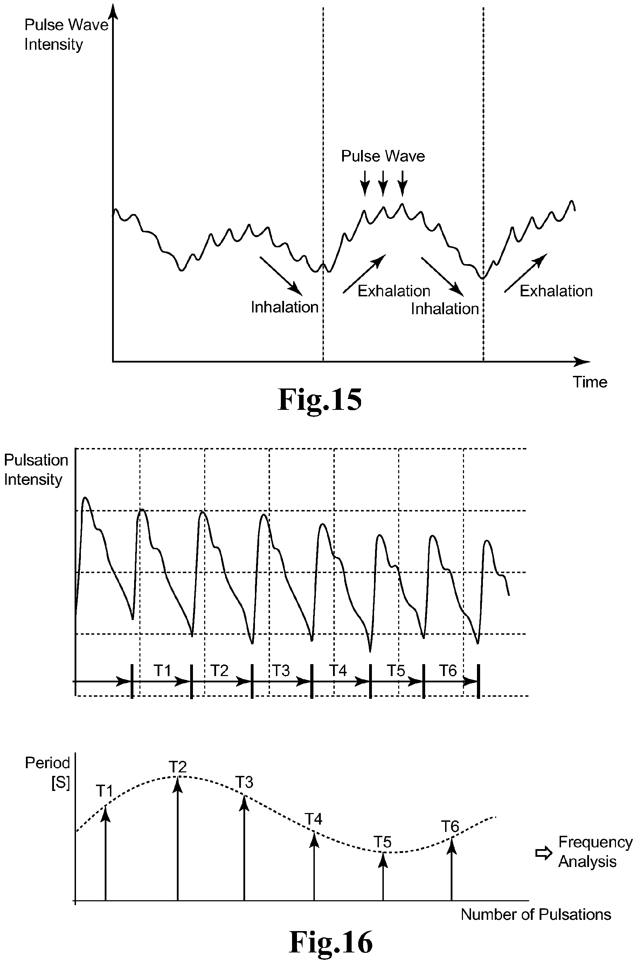

{Fig. 1} Fig. 1 is a diagram illustrating an outline of a biological information reading device according to a first embodiment of the present invention.
{Fig. 2} Fig. 2 is a block diagram of the biological information reading device according to the first embodiment of the present invention.
{Fig. 3} Fig. 3 is a diagram illustrating an example of an implementation form of the biological information reading device of Fig. 2.
{Fig. 4} Fig. 4 is a diagram illustrating a state of incident light and reflected light when a refractive index of air is different from a refractive index of a living body as a Comparative Example.
{Fig. 5} Fig. 5 is a diagram illustrating a state of incident light when a refractive index of an adhesive layer and a quarter-wave plate is equal to a refractive index of a living body in the biological information reading device according to the first embodiment of the present invention.
{Fig. 6} Fig. 6 is a diagram illustrating a modified example of the biological information reading device according to the first embodiment of the present invention.
{Fig. 7} Fig. 7 is a diagram illustrating a pulse wave form of actual superficial temporal artery.
{Fig. 8} Fig. 8 is a diagram for description of a method of determining an attachment place of the biological information reading device according to the first embodiment of the present invention.
{Fig. 9} Fig. 9 is a diagram illustrating guide sound volume and quantity of light at the time of detecting a blood vessel in the method of determining the attachment place of Fig. 8.
{Fig. 10} Fig. 10 is a flowchart illustrating an operation of an operation unit of Fig. 2.
{Fig. 11} Fig. 11 is a block diagram of a biological information reading device according to a second embodiment of the present invention.
{Fig. 12} Fig. 12 is a block diagram of a biological information reading device according to a third embodiment of the present invention.
{Fig. 13} Fig. 13 is a block diagram of a biological information reading device according to a fourth embodiment of the present invention.
{Fig. 14} Fig. 14 is a diagram illustrating an example of an implementation form of a biological information reading device according to a fifth embodiment of the present invention.
{Fig. 15} Fig. 15 is a diagram illustrating a state in which a low frequency component overlaps a pulse wave in connection with respiration.
{Fig. 16} Fig. 16 is a diagram for description of a method of analyzing a fluctuation frequency in a variation in which periods of a pulse wave form are arranged in time series.

### {Description of Embodiments}

Prior to describing embodiments in detail, terms used in this specification will be defined.

In this specification, for example, a term "biological information" refers to information obtained by a measurement method conforming to a definition as information regarding to a state of a living body in which a blood pressure by invasive arterial blood pressure measurement is 110 mmHg or a blood glucose level by glucose analyzer (POCT) is 120 mg/dl, or information obtained by a method widely recognized as a practical standard in a medical world such as a cuff-type sphygmomanometer.

Note that, besides a blood pressure, types of the biological information include blood flow volume, a blood flow rate, a blood component (a blood glucose level, an ion, or the like), blood oxygen concentration, a body temperature, a heart rate, a cardiac cycle, a breathing rate, a breathing cycle, an autonomic nerve activity ratio, a pulse wave propagation velocity, an expansion/contraction ratio of a capillary vessel, a relaxation/stiffness ratio of muscles, a lactic acid accumulation ratio, a sweat rate, a state of activity (exercise/rest/sleep), a reaction to external stimulus (a temperature change or the like), and the like.

In this specification, a term "biological signal" refers to a signal obtained by a living body, and includes an emission from the living body such as respiratory sound, cardiac electricity, myoelectricity, or a brain wave, or a passive reaction to external energy such as ultrasonic echo or reflected light.

In this specification, a term "estimated biological information" refers to biological information serving as an estimated value obtained by performing arithmetic processing based on the biological signal.

### [First embodiment]

A description will be given of an outline of a biological information reading device 1 according to an embodiment of the present invention with reference to Fig. 1.

As illustrated in Fig. 1, the biological information reading device 1 is attached to a part of a skin 40 (a surface of a living body) of an object to be measured corresponding to a measured person suitable for acquisition of a pulsation of a blood vessel to acquire a blood vessel pulsation waveform as living body measurement information of the object to be measured by a biological information acquisition unit 2 using an optical sensor. The biological information acquisition unit 2 includes a projector 3 and an optical receiver 4 as the optical sensor. Note that, the projector 3 and the optical receiver 4 illustrated in Fig. 1 have different forms from those illustrated in Fig. 2 described below. However, Fig. 1 is a diagram for a conceptual description of the whole reading of the blood vessel pulsation waveform, and the projector 3 and the optical receiver 4 are conceptually illustrated. Note that, the blood pressure estimation method disclosed in PTL 1 is highly compatible with the present embodiment, and the same blood pressure estimation method as that of PTL 1 may be used in the present embodiment.

The biological information reading device 1 illustrated in Fig. 1 includes a pulsation detector 20 connected to the biological information acquisition unit 2 to output a pulsation waveform from emitted light and received light, a blood pressure estimation unit 21 that estimates a blood pressure value from pulsation waveform data, an alert issuing unit 22 that issues an alert based on an estimation result of the blood pressure estimation unit 21, and an output unit 23 that sounds or displays an alarm. Note that, the pulsation detector 20, the blood pressure estimation unit 21, and the alert issuing unit 22 are collectively referred to as an operation unit 24.

The biological information reading device 1 according to a first embodiment of the present invention will be described below. As illustrated in Fig. 2, the biological information reading device 1 according to the present embodiment is used by attaching a portion corresponding to the biological information acquisition unit 2 illustrated in Fig. 1 to a surface (skin) of a human body, and allows a pulse, a blood pressure, and the like of a measured person to be continuously monitored in a noninvasive state. Further, in the biological information reading device 1, the operation unit 24 and the output unit 23 illustrated in Fig. 1 are configured integrally with the biological information acquisition unit 2 attached to the surface (skin) of the human body. The biological information reading device 1 is provided in a form that allows attachment to the human body surface as described above. Hereinafter, details thereof will be described.

The biological information reading device 1 includes a light emitting element 10 serving as a polarized light emitter, a polarizing plate 11, a light receiving element 12 serving as a light receiver, a quarter-wave plate 13 serving as a polarization property change portion, a permeable membrane 14 serving as a permeable membrane portion, a pulsation detector 20, a blood pressure estimation unit 21, an alert issuing unit 22, an output unit 23, and a battery 25. Note that, the light emitting element 10 and the quarter-wave plate 13 illustrated in Fig. 2 correspond to the projector 3 illustrated in Fig. 1, and the quarter-wave plate 13, the polarizing plate 11, and the light receiving element 12 correspond to the optical receiver 4 illustrated in Fig. 1.

That is, portions corresponding to the biological information acquisition unit 2 are the light emitting element 10, the polarizing plate 11, the light receiving element 12, the quarter-wave plate 13, and the permeable membrane 14. Note that, the pulsation detector 20, the blood pressure estimation unit 21, and the alert issuing unit 22 are collectively referred to as the operation unit 24. In addition, even though wiring of the battery 25 is omitted in Fig. 2 and Fig. 3, the battery 25 supplies power to the light emitting element 10, the light receiving element 12, and the operation unit 24. In drawings below, the above description with regard to the wiring of the battery 25 is similarly applied.

The light emitting element 10 emits linearly polarized light as outward light 30. For example, a polarization direction of the outward light 30 is a direction along a short direction of the permeable membrane 14 illustrated in Fig. 3. One example of the light emitting element 10 is a surface light emitting body such as an organic light emitting diode (LED), which may be implemented a linearly polarized light emitting function by processing in a manufacturing process.

When an organic LED is used as the light emitting element, there is an advantage in that heat generating places associated with light emission are not concentrated unlike other LEDs since the organic LED is a surface light emitting body. Further, even when the organic LED is continuously used for a long time, there is a less concern that a skin is burned. Thus, the organic LED is suitable for measurement continued for a long time.

Herein, a used wavelength region of light will be described. As a wavelength region of light emitted by the light emitting element 10, a range of 700 nm to 900 nm may be used as a near-infrared light region, and the vicinity of 450 nm and the vicinity of 520 nm may be used in a visible light region.

Near-infrared light penetrates the living body more than visible light or mid/far-infrared light does, and thus may reach a deep portion under the skin. In addition, near-infrared light is absorbed by hemoglobin in blood at a certain absorbance. An absorbance of hemoglobin has a significant difference when compared to a scattering rate of a subcutaneous tissue. Thus, when the skin is irradiated with near-infrared light, and light reflected/diffused under the skin is detected, a change in hemoglobin value under the skin may be perceived.

In a part near a wrist (palmar side) in which artery is at a shallow position under the skin, physical vibration due to pulsation of the artery strongly affects a capillary vessel under the skin, and in this way, the hemoglobin value under the skin greatly varies. In addition, in a part such as a finger tip in which the density of capillary vessels under the skin is high, pulsation of the artery strongly affects the hemoglobin value under the skin.

In these parts, the hemoglobin value under the skin varies in synchronization with the pulse wave, and thus the pulse wave may be detected from the variation of the hemoglobin value under the skin.

In addition, a light ray corresponding to a visible ray having a wavelength of around 450 nm (blue) or around 520 nm (green) has a characteristic in a light absorption characteristic of hemoglobin or bilirubin in blood, can detect a variation in hemoglobin value under the skin when reflection/diffusion characteristics of skin tissues, and the like are combined, and thus can be used to measure the pulsation.

The polarizing plate 11 is effective in transmitting only light linearly polarized in a certain direction. A polarization direction in which the polarizing plate 11 can transmit light is provided to be different from linearly polarized light emitted by the light emitting element 10 by 90 degrees. For this reason, even though the light emitting element 10 emits light in a direction other than a direction of the outward light 30 (for example, a direction toward the light receiving element 12), polarized light in the direction toward the light receiving element 12 is shielded by the polarizing plate 11 and cannot arrive at the light receiving element 12. In this way, light reflected on a surface of the skin may be excluded, and light diffused and reflected under the skin may be defected by the light receiving element 12.

The light receiving element 12, for example, a photodiode, is an element that receives light penetrating the polarizing plate 11, and generates an electric signal of a voltage depending on strength of the received light. However, for example, the light receiving element 12 may be a light receiving element using an organic thin film material such as an organic complementary metal-oxide semiconductor (CMOS) sensor. In particular, the organic thin film material is preferably formed using a flexibly deformable material similar to an organic LED described below. The light receiving element 12 converts strength of received light into an electric signal, and outputs the electric signal to the pulsation detector 20. Note that, the light receiving element 12 does not have a polarization property.

The quarter-wave plate 13 is disposed to change the outward light 30 of the linearly polarized light emitted by the light emitting element 10 to light circularly polarized clockwise. Further, the quarter-wave plate 13 changes reflected light 31 reflected inside the skin 40 and circularly polarized counterclockwise to linearly polarized light, which is set to return light 32. As a result, the outward light 30 and the return light 32 are linearly polarized lights, polarization directions of which are different from each other by 90 degrees.

One surface side of the permeable membrane 14 attached to the skin 40 corresponds to a transparent or translucent sheet to which an adhesive for attaching the permeable membrane 14 to the skin 40 is applied. The permeable membrane 14 is made of a flexibly deformable material to conform to a shape of a surface of a human body. Examples of the permeable membrane 14 include various transparent resin films. Note that, for example, it is possible to use various high moisture permeable transparent films including a polystyrene film, a polyurethane elastomer film, and the like in view of evaporating moisture such as sweat or the like from the human body surface.

In addition, the quarter-wave plate 13 and the light emitting element 10, the polarizing plate 11, and the light receiving element 12 connected thereto are disposed on the other surface side of the permeable membrane 14 opposite to the skin 40. Note that, the quarter-wave plate 13 is directly attached to the permeable membrane 14, and the permeable membrane 14 and the quarter-wave plate 13 are fixed to each other using a transparent adhesive and the like. Therefore, the quarter-wave plate 13 may be peeled off from the permeable membrane 14, and the quarter-wave plate 13 may be attached to a new permeable membrane 14 again. In this way, the permeable membrane 14 having a decreased adhesive force with respect to the skin 40 may be replaced with a new permeable membrane 14.

The light emitting element 10, the polarizing plate 11, the light receiving element 12, and the quarter-wave plate 13 are preferably made of a flexibly deformable material. A plate having a synthetic resin property such as deformable and flexible polycarbonate or the like is provided as the polarizing plate 11 and the quarter-wave plate 13, and thus the polarizing plate 11 and the quarter-wave plate 13 may be used. A deformable organic LED may be used as the light emitting element 10, and a deformable organic CMOS element may be used as the light receiving element 12. However, when there is a difficulty in assigning a flexibly deformable property to these respective parts, these respective parts may be compactly provided with respect to the permeable membrane 14. That is, when the light emitting element 10, the polarizing plate 11, the light receiving element 12, and the quarter-wave plate 13 occupy a small area with respect to the permeable membrane 14, poor conformability of these respective parts may be compensated for when the permeable membrane 14 is flexibly deformed by conforming to the human body surface, and thus, it is possible to prevent the biological information reading device 1 from being poorly attached to the human body surface.

A description will be given of an effect of employing an adhesive plaster type in which the light emitting element 10, the polarizing plate 11, the light receiving element 12, the quarter-wave plate 13, and the permeable membrane 14 are stacked, and the permeable membrane 14 is used as an adhesive layer as described above. A/D conversion needs to be accurately performed within a relatively narrow input range in order to acquire a pulse wave form by irradiation light and return light thereof. Thus, it is desirable that an adhesive state of a measurement element on a skin surface be stable. There is a measurement element which has flexibility and has no plasticity. The skin is flexible and has flexibility and plasticity, and thus the measurement element may be peeled off from the skin without deformation of the measurement element being able to conform to a flexible change of the skin. When the permeable membrane 14 coming into contact with the skin is interposed as an adhesive layer between the measurement element and the skin, a greater deformation degree is allowed with respect to deformation of the skin, a degree of freedom of an installation part is improved, and measurement may be continuously performed at all times. In addition, installation is allowed in an exercise condition, and thus biological information in the exercise condition may be measured.

In addition, since the skin 40 and air have different refractive indices, about 4% of reflection is expected to occur when air enters between the light emitting element 10 and the skin 40. On the other hand, not interposing air between the light emitting element 10 and the skin 40 as illustrated in Fig. 2 is useful for reducing loss of a biological signal. For example, since a refractive index of the skin 40 is about 1.5, when refractive indices of the quarter-wave plate 13 and the permeable membrane 14 are set to about 1.5 accordingly, loss of the biological signal may be minimized.

That is, when air, a refractive index of which is 1.0, is interposed between a light source and the living body (skin 40), a refractive index of which is about 1.5, as illustrated in Fig. 4, about 4% of reflected light is generated with respect to incident light from the light source. On the other hand, when the permeable membrane 14 and the quarter-wave plate 13, refractive indices of which are 1.5, are interposed between the projector 3 and the living body (skin 40) as illustrated in Fig. 5, incident light from the projector 3 may arrive at the skin 40 without most of the incident light being reflected.

In addition, when the permeable membrane 14 does not transmit light in a part other than a part in which the quarter-wave plate 13 is disposed, invasion of ambient light may be prevented. For example, lightproof paint is preferably applied to the part other than the part in which the quarter-wave plate 13 is disposed.

In addition, as illustrated in Fig. 6, the biological information reading device 1 may be configured by covering an opposite side from a contact surface of the biological information acquisition unit 2 coming into contact with the skin 40 with a permeable membrane 14a. In this case, forming the whole permeable membrane 14a using a lightproof material is preferable in preventing invasion of ambient light. Shielding ambient light such as indoor light, sunlight, or the like is effective in improving signal to noise ratio and increasing detection accuracy.

In addition, with regard to a part of the skin 40 in which the biological information reading device 1 is installed, for example, when a brain disease is predicted, it is preferable to measure a pulse wave at a portion around external carotid artery corresponding to artery that supplies blood flow to a brain or branch artery thereof. Examples thereof include superficial temporal artery, facial artery, occipital artery, posterior auricular artery, ascending pharyngeal artery, zygomatico-orbital artery, and the like. In this way, a pulse wave form serving as a more realistic influence on the brain may be obtained, and accuracy is further increased.

For example, Fig. 7 illustrates pulse wave strength obtained when the measured person bends forward and pulse wave strength obtained when the measured person stands up at superficial temporal artery. Fig. 7 is a diagram in which a horizontal axis represents time and a vertical axis represents pulse wave strength. As shown in Fig. 7, a great variation in pulse wave is seen in superficial temporal artery only when the measured person bends forward after standing up. Therefore, it can be understood that a variation in pulse wave form of the measured person is precisely perceived when the biological information reading device 1 is installed in superficial temporal artery.

The pulsation detector 20 is an information processing device that detects pulsation based on a result of a comparison between the outward light 30 emitted by the light emitting element 10 and the return light 32 received by the light receiving element 12. Note that, although not illustrated, it is possible to employ a configuration in which a change-over switch is provided to switch between a wire reaching the output unit 23 from the pulsation detector 20 through the blood pressure estimation unit 21 and the alert issuing unit 22 and a wire that directly connects the pulsation detector 20 to the output unit 23 in Fig. 2, and the output unit 23 switches to a mode in which a sound signal, a flash signal, or the like is output depending on a detection result of the pulsation detector 20. In this way, when the biological information reading device 1 is temporarily placed on the skin 40, and the detection result of the pulsation detector 20 is recognized by a signal output from the output unit 23, a part of the skin 40 in which pulsation is easily detected may be easily located. For example, the biological information reading device 1 is temporarily placed on a portion around external carotid artery or branch artery thereof described above, and whether pulsation is actually favorably detected is determined using the signal output from the output unit 23. When the pulsation is favorably detected, the biological information reading device 1 is attached to a part thereof. In this way, it is possible to easily and reliably determine an optimal attachment position of the biological information reading device 1.

For example, when the biological information reading device 1 is allowed to pass above radial artery of a left wrist as illustrated in Fig. 8, guide sound volume or quantity of light rapidly increases at the time of passing above a blood vessel as illustrated in Fig. 9. In this way, it is possible to specify a position of the radial artery, and precisely install the biological information reading device 1.

The blood pressure estimation unit 21 is an information processing device that estimates a blood pressure based on pulsation detected by the pulsation detector 20. Note that, an estimation method based on periodic pulsation waveform data described in PTL 1 is used as a method of estimating a blood pressure using the pulsation detector 20 and the blood pressure estimation unit 21.

Further, a correlation between a pulsation waveform and pressure variation data inside a blood vessel 41 may be corrected using a scheme disclosed in PTL 2. That is, an estimated blood pressure value obtained by the biological information reading device 1 may be compared with a measured blood pressure value using a conventional cuff. When there is a gap between the values, the estimated blood pressure value obtained by the biological information reading device 1 may be corrected to reduce the gap. Note that, in this instance, information needs to be exchanged by connecting a sphygmomanometer using the cuff to the biological information reading device 1. It is preferable that an individual identifier be assigned to the biological information reading device 1, the identifier be stored in a memory (not illustrated) inside the biological information reading device 1, and the identifier be assigned to information when the information is transmitted to and received from the sphygmomanometer. In this way, one sphygmomanometer may individually respond to a plurality of biological information reading devices 1 by identifying the respective biological information reading devices 1.

The alert issuing unit 22 issues an alert when a blood pressure estimated by the blood pressure estimation unit 21 is out of a range of a normal value.

In response to receiving an output of the alert of the alert issuing unit 22, the output unit 23 reports issue of the ert to the outside using sound, light, or the like. When the alert corresponds to sound, for example, the output unit 23 is a small speaker, a sounducer, or the like. In addition, when the alert corresponds to light, for example, the output unit 23 is a light emitting diode or the like.

Note that, when the biological information reading device 1 is mounted on the skin 40, the biological information reading device 1 is preferably mounted after confirming that a blood pressure value of the measured person corresponds to a normal value. In this way, at the time of mounting the biological information reading device 1, when a alert is issued since the biological information reading device 1 improperly mounted, the fact may be noticed.

The battery 25 supplies power to the light emitting element 10, the light receiving element 12, and the operation unit 24. For example, the battery 25 is a lithium battery referred to as a button battery.

A mounting state of the light emitting element 10, the polarizing plate 11, the light receiving element 12, the quarter-wave plate 13, the permeable membrane 14, the operation unit 24 (the pulsation detector 20, the blood pressure estimation unit 21, and the alert issuing unit 22), the output unit 23, and the battery 25 described above is illustrated in Fig. 3. The quarter-wave plate 13 is disposed on the adhesive plaster-shaped permeable membrane 14. Further, the light emitting element 10, the polarizing plate 11, and the light receiving element 12 are overlapped and disposed on the quarter-wave plate 13. Furthermore, a portion of an inside of the quarter-wave plate 13, the light emitting element 10, the polarizing plate 11, and the light receiving element 12 is hollowed out, and the operation unit 24 including the pulsation detector 20, the blood pressure estimation unit 21, and the alert issuing unit 22 and the battery 25 are mounted therein. In addition, the output unit 23 is mounted on an upper portion of the biological information reading device 1. Note that, the biological information reading device 1 does not have a power switch, and is configured to be turned ON and operated by mounting the battery 25. The battery 25 may be mounted immediately before a user uses the biological information reading device 1.

Next, an operation of the operation unit 24 will be described with reference to a flowchart of Fig. 10. A condition of START of the flowchart of Fig. 10 is a condition that the battery 25 is mounted in the biological information reading device 1, and the biological information reading device 1 operates. In addition, processing from START to END in the flowchart of Fig. 10 is processing corresponding to one cycle. When processing corresponding to one cycle ends, and the condition of START is satisfied, processing starts again.

In step S1, the pulsation detector 20 of the operation unit 24 determines whether a pulse wave could be acquired based on an output of the light receiving element 12. When it is determined that the pulse wave could be acquired in step S1, the operation proceeds to step S2. On the other hand, when it is determined that the pulse wave cannot be acquired in step S1, the operation of step S1 is repeated.

In step S2, the blood pressure estimation unit 21 of the operation unit 24 estimates a blood pressure from pulsation information acquired by the pulsation detector 20 using, for example, the above-described method disclosed in PTL 1. When the blood pressure is estimated in step S2, the operation proceeds to S3.

In step S3, the alert issuing unit 22 of the operation unit 24 determines whether the blood pressure estimated by the blood pressure estimation unit 21 falls within a range of a normal value. When the blood pressure is determined to fall within the range of the normal value in step S3, the operation ends processing corresponding to one cycle (END). On the other hand, when the blood pressure is determined to be out of the range of the normal value, the operation proceeds to step S4.

The alert issuing unit 22 of the operation unit 24 instructs the output unit 23 to output an alert in step S4, and the operation ends processing corresponding to one cycle (END).

As described above, the biological information reading device 1 may emits polarized light as the outward light 30, allows the outward light 30 to enter the inside of the skin 40, and receives the return light 32, which has a different polarization property from that of the outward light 30 and returns by being reflected in the inside of the skin 40, thereby detecting a change in pulsation depending on a phase difference between the outward light 30 and the return light 32 (step S1 of Fig. 10). Further, for example, the biological information reading device 1 may previously obtain and store a correlation between sampling data of a pulsation waveform and pressure variation data inside the blood vessel 41 according to an invasive method and the like, thereby estimating a blood pressure from a pulsation detection result (step S2 of Fig. 10), and issue an alert depending on a blood pressure estimation result (step S4 of Fig. 10).

According to the biological information reading device 1, it is possible to mount the device on the measured person using a scheme in which the adhesive plaster is attached to the skin, and to acquire information about a pulse wave or a blood pressure of the measured person at all times. For example, according to the biological information reading device 1, it is possible to employ a system in which, when a blood pressure has an abnormal value based on blood pressure information of the measured person, the abnormality is reported to the measured person or a person around the measured person at all times. Further, it is possible to detect an abnormal blood pressure of the measured person in early stage.

Simple determination based on a high or low blood pressure value has been described in the flowchart of Fig. 10. However, with regard to a serious disease, whether to issue an alert is determined by combining a plurality of variations in biological information besides the blood pressure value.

An estimated biological information time series storage unit, a determination unit, or a determination information holding unit (not illustrated) may be provided in the operation unit 24. The estimated biological information time series storage unit has a function of storing obtained estimated biological information in time series. The determination unit identifies an indication of a particular disease by performing a time-series analysis of the estimated biological information stored in the estimated biological information time series storage unit. The determination information holding unit holds a method of making a determination based on the estimated biological information stored in the estimated biological information time series storage unit to identify an indication of a particular disease. When the determination unit is software operated by a central processing unit (CPU), determination information held in the determination information holding unit corresponds to an algorithm.

For example, the determination information is based on a combination of information such as an age, a gender, a height, a weight, a body fat percentage, a body water percentage, a previous history, whether medicine is taken, an arteriosclerosis level, a skin color, or female menopause of the measured person.

When the estimated biological information time series storage unit is included, a rapid change in biological information for a short time may be detected, and a measure may be taken immediately before a serious state. The estimated biological information stored in the estimated biological information time series storage unit may be transmitted to the outside, and analyzed in time series using another external device.

### {Second embodiment}

A description will be given of a biological information reading device 1a according to a second embodiment of the present invention with reference to Fig. 11. The biological information reading device 1 is partially different from the biological information reading device 1a of the present embodiment. Therefore, the same or a similar reference numeral as or to that of the biological information reading device 1 will be assigned to the same member as that of the biological information reading device 1.

A light receiving element 12a of the biological information reading device 1a is a polarized light receiver having a polarization property in sensitivity thereof. The polarization property of the light receiving element 12a is different from a polarization property of the outward light 30 emitted by the light emitting element 10. For example, a direction of linearly polarized light of the outward light 30 is different from a direction of linearly polarized light of the light receiving element 12a by 90 degrees.

In the biological information reading device 1a, the projector 3 illustrated in Fig. 1 corresponds to a light emitting element 10 and a quarter-wave plate 13, and the optical receiver 4 corresponds to the quarter-wave plate 13 and the light receiving element 12a.

According to the biological information reading device 1a, the polarizing plate 11 necessary in the biological information reading device 1 may be omitted. That is, even though polarized light from the light emitting element 10 directly arrives at the light receiving element 12a, a polarization property of the polarized light is different from a polarization direction in which the light receiving element 12a has sensibility, and thus has no influence.

Accordingly, the biological information reading device 1a may be further miniaturized and lightened when compared to the biological information reading device 1.

### {Third embodiment}

A description will be given of a biological information reading device 1b according to a third embodiment of the present invention with reference to Fig. 12. The biological information reading device 1 is partially different from the biological information reading device 1b of the present embodiment. Therefore, the same or a similar reference numeral as or to that of the biological information reading device 1 will be assigned to the same member as that of the biological information reading device 1.

In the biological information reading device 1b, the projector 3 illustrated in Fig. 1 corresponds to a light emitting element 10 and a quarter-wave plate 13, and the optical receiver 4 corresponds to the quarter-wave plate 13, a polarizing plate 11a, and a light receiving element 12c.

The biological information reading device 1b is different from the biological information reading device 1 in that the biological information reading device 1b includes a light receiving element 12b for measuring quantity of light of the light emitting element 10.

When the biological information reading device 1b includes the light receiving element 12b for measuring quantity of light, it is possible to detect a change in quantity of light of the light emitting element 10. For example, a voltage of a battery 25 decreases from an initial voltage as a use time increases. With a voltage drop of the battery 25, quantity of light of the light emitting element 10 decreases. In this instance, when the biological information reading device 1b includes the light receiving element 12b for measuring quantity of light, a correction may be performed such that light receiving sensitivity of the light receiving element 12c is increased in response to detection of a decrease in quantity of light of the light emitting element 10, and the decrease in quantity of light of the light emitting element 10 may be compensated for. According to the above-described configuration of the biological information reading device 1b, a pulsation detector 20 may receive output information from the light receiving element 12c under the same condition at all times even when a voltage drop of the battery 25 is generated. Accordingly, high detection accuracy for pulsation in the pulsation detector 20 may be maintained.

### {Fourth embodiment}

A description will be given of a biological information reading device 1c according to a fourth embodiment of the present invention with reference to Fig. 13. The biological information reading device 1 is partially different from the biological information reading device 1c of the present embodiment. Therefore, the same or a similar reference numeral as or to that of the biological information reading device 1 will be assigned to the same member as that of the biological information reading device 1.

The biological information reading device 1c includes a radio signal transmitter 25, which transmits a blood pressure estimation result of a blood pressure estimation unit 21 as a radio signal, in an operation unit 24a. Further, the biological information reading device 1c includes a radio signal receiver 26, which receives the radio signal transmitted by the radio signal transmitter 25, separated from the operation unit 24a. An output of the radio signal receiver 26 corresponds to the blood pressure estimation result of the blood pressure estimation unit 21, and is input to an alert issuing unit 22a connected to the radio signal receiver 26. An output unit 23 is connected to the alert issuing unit 22a.

As described above, the biological information reading device 1c includes the radio signal receiver 26, the alert issuing unit 22a, and the output unit 23 disposed separately from the operation unit 24a, and thus may recognize an issued alert at a place separated from the measured person. For example, when a separate unit (the radio signal receiver 26, the alert issuing unit 22a, and the output unit 23) is configured in a form such as an ear hanging type or ear hole type hearing aid, and the hearing aid is worn in an ear of the measured person, the measured person does not fail to hear the alert. The alert issuing unit may have flexibility by using a flexible piezoelectric film speaker in the alert issuing unit, and have a configuration that allows deformation of the unit along a shape of an opening of the ear. Alternatively, when the biological information reading device 1c is used for an inpatient inside a hospital, the separate unit may be installed in a nurse station or the like, thereby monitoring an abnormal blood pressure of the inpatient in the nurse station or the like at all times. Further, when the device is worn by a measured person who exercises, biological information in an exercise condition may be acquired at a separate place.

Besides, when communication via a network such as the Internet or the like is allowed between the radio signal transmitter 25 and the radio signal receiver 26, the separate unit may be installed at a remote location. For example, the biological information reading device 1c may be installed on a senior citizen who lives alone, and the separate unit may be installed in a house of a family or the like at a remote location.

In addition, when compared to the biological information reading devices 1, 1a, and 1b in the above-described first to third embodiments, components in a portion installed in (attached to) a human body decreases, and thus the portion installed in (attached to) the human body may be lightened. Note that, when focusing on a weight reduction of the portion installed in the human body, light reception information of a light receiving element 12 may be transmitted from the radio signal transmitter 25 using a radio signal. In this case, in the separate unit, all components of the operation unit 24 (a pulsation detector 20, the blood pressure estimation unit 21, and the alert issuing unit 22a) and the output unit 23 are disposed on a side of the radio signal receiver 26. According to the above-described configuration of the biological information reading device 1c, a weight reduction on a side at which the portion is installed in the human body may be attempted.

### {Fifth embodiment}

A description will be given of a biological information reading device 1d according to a fifth embodiment of the present invention with reference to Fig. 14. The biological information reading device 1d of the present embodiment has a configuration in which a plurality of biological information reading units 1e is disposed on one permeable membrane 14a. The biological information reading units 1e are obtained by excluding the permeable membrane 14 from the biological information reading devices 1, 1a, and 1b.

According to the biological information reading device 1d having the above-described configuration, the plurality of biological information reading units 1e simultaneously measures substantially the same part of the same measured person, and thus it is possible to improve measurement accuracy and reliability.

Further, as an example, and as described in NPL 1, with regard to arteriosclerosis closely connected with a blood pressure, when two sensors with a predetermined interval are disposed inside the same device, and a propagation velocity is calculated based on a delay time of pulse waves, a pseudo-test of a pulse wave velocity (PWV) corresponding to an indicator of arteriosclerosis may be conducted. Therefore, according to the biological information reading device Id, it is possible to conduct a pseudo-test of a PWV corresponding to an indicator of arteriosclerosis by disposing the plurality of biological information reading units 1e with predetermined intervals on the one permeable membrane 14a, and calculating a propagation velocity based on a delay time of pulse waves.

### {Other embodiments}

Each of the pulsation detector 20, the blood pressure measurement unit 21, and the alert issuing unit 22 has an information processing unit. However, functions thereof may be implemented in one information processing unit. That is, the information processing unit may implement the pulsation detector 20, the blood pressure measurement unit 21, and the alert issuing unit 22 excepting the output unit by executing a predetermined program installed in advance. For example, the information processing unit includes a memory, a CPU, an input/output port, and the like. The CPU of the information processing unit reads a control program as a predetermined program from the memory or the like, and executes the control program. In this way, functions of the pulsation detector 20, the blood pressure measurement unit 21, and the alert issuing unit 22 excepting the output unit are implemented in the information processing unit. Note that, instead of the CPU, it is possible to use an application specific integrated circuit (ASIC), a microprocessor (microcomputer), a digital signal processor (DSP), or the like.

In addition, the above-described predetermined program may be stored in the memory of the information processing unit or the like before shipment of the pulsation detector 20, the blood pressure measurement unit 21, and the alert issuing unit 22, and may be stored in the memory of the information processing unit or the like after shipment of the pulsation detector 20, the blood pressure measurement unit 21, and the alert issuing unit 22. Alternatively, a portion of the program may be stored in the memory of the information processing unit or the like after shipment of the pulsation detector 20, the blood pressure measurement unit 21, and the alert issuing unit 22. For example, the program stored in the memory of the information processing unit or the like after shipment of the pulsation detector 20, the blood pressure measurement unit 21, and the alert issuing unit 22 may be obtained by installing a program stored in a computer-readable recording medium such as a CD-ROM or the like, and may be obtained by installing a program downloaded through a transmission medium such as the Internet or the like.

In addition, the above-described predetermined program includes a program executable by being installed in a hard disk and the like in addition to a program directly executable by the information processing unit. Further, the program includes a compressed or encrypted program.

As described above, when the functions of the pulsation detector 20, the blood pressure measurement unit 21, and the alert issuing unit 22 excepting the output unit are implemented by the information processing unit and the program, it is possible to flexibly respond to mass production or specification change (or design change).

Note that, the program executed by the information processing unit may be a program processed in time series along a sequence described in the present specification, or a program processed in parallel or at a necessary time such as a time at which a call is performed.

In the above-described embodiments, the organic LED is given as an example of the light emitting element 10. However, the light emitting element 10 is not restricted thereto. For example, a light emitting diode corresponding to surface light emission or the like may be used as the light emitting element 10.

In addition, in the above-described embodiments, a description has been given of a case in which the organic LED is used as the light emitting element 10. However, for example, in an environment such as a human body surface in which a lot of moisture is given, an organic material such as the organic LED easily deteriorates, and deteriorates due to oxygen. In this regard, when the light emitting element 10 is the organic LED, it is possible to employ a configuration in which the whole light emitting element 10 or a main part thereof is covered with a protective layer to protect the light emitting element 10 from moisture or oxygen.

In addition, the above-described embodiments have described that pulsation is detected by a phase difference between the outward light 30 and the return light 32. However, furthermore, pulsation may be detected depending on various comparison results between the outward light 30 and the return light 32. For example, the outward light 30 is light having a wavelength in which the light is easily absorbed by blood, and thus the absorbed amount of light is different between a case in which the amount of blood flow inside the blood vessel 41 is large and the amount is small. Therefore, a change in pulsation may be detected depending on a result of a comparison between intensity of the return light 32 and intensity of the outward light 30.

In addition, although not illustrated, in Fig. 2, in addition to the wire reaching the output unit 23 from the pulsation detector 20 through the blood pressure estimation unit 21 and the alert issuing unit 22, the wire that directly connects the pulsation detector 20 to the output unit 23 may be provided, and the output unit 23 may output a sound signal, a flash signal, or the like depending on a detection result of the pulsation detector 20. In this way, when the detection result of the pulsation detector 20 is recognized by a signal output from the output unit 23, for example, an alert may be issued with regard to a rise in pulse rate corresponding to a sign of a rise in blood pressure. That is, the measured person may be aware of a sign of a rise in blood pressure using a rise in pulse rate before receiving an alert against the rise in blood pressure. In this way, the measured person may take an action to avoid the rise in blood pressure. As described above, when the alert is set in two stages, the measured person may take appropriate measures before a serious state.

In addition, Fig. 15 is a diagram in which a horizontal axis represents time, and a vertical axis represents intensity of a pulse wave. As illustrated in Fig. 15, it is known that a low frequency component overlaps a pulse wave in connection with respiration. For example, the blood pressure estimation unit 21 may increase accuracy of blood pressure estimation by separating a low frequency component from pulsation detected by the pulsation detector 20. Further, a respiration state of the measured person may be identified by separating a low frequency component from pulsation detected by the pulsation detector 20. For example, it is possible to additionally provide a function of monitoring an active state of a sympathetic nerve and a parasympathetic nerve depending on a respiration state of the measured person.

Further, as illustrated in Fig. 16, an active state of a sympathetic nerve and a parasympathetic nerve can be identified by analyzing a fluctuation frequency in a variation in which periods of a pulse wave form are arranged in time series, and thus a determination function using this fact may be additionally provided. In an upper diagram of Fig. 16, a horizontal axis represents time, and a vertical axis represents pulsation intensity of a pulse wave. In a lower diagram of Fig. 16, a horizontal axis represents the number of pulsations, and a vertical axis represents a period (S: second).

More specifically, first, a filter using a differential circuit is applied to a pulsation waveform signal, and a zero crossing point is detected. In this way, it is possible to identify a peak position immediately after a rise of the pulsation waveform signal. A period of pulsation is obtained when peak positions are continuously acquired. In Fig. 16, time represented in from T1 to T6 refers to period time of each waveform.

Frequency analysis is performed to identify the form of a change (=fluctuation) in a pulsation level of a value of a period Tn successively obtained as described above, and an active state of a sympathetic nerve and a parasympathetic nerve can be identified using a fluctuation frequency obtained in this way. The above description is similarly applied to a scheme of a time component index such as a Lorentz plot evaluated by an interval from a previous heart beat for each heart beat.

In addition, it is possible to determine whether a sleeping state is entered, quality of sleep, or the like based on pulsation, respiration, an active state of a sympathetic nerve, or the like. In this way, it is possible to identify sleep apnea syndrome and the like.

With regard to an alert when abnormality is present as a result of making a determination regarding acquired biological information, a notification is provided to the measured person using alarm display (sense of sight} or alarm sound (auditory sense). However, the present invention is not restricted thereto. For example, it is possible to use a particular vibration pattern (sense of touch), spray of fragrance liquid (olfactory sense), control of a stimulation liquid discharge device installed in a mouth in advance (sense of taste), and the like.

Additionally, a method other than a method of issuing an alert is present with regard to a response to a case in which abnormality is present as a result of making a determination regarding acquired biological information. It is natural to inform the measured person or a third party that abnormality is present. However, for example, when a portable or buried liquid medicine injection device is previously installed at all times in order to treat a particular disease, the liquid medicine injection device may be controlled to start operating. For example, when an angina symptom is detected from biological information, a device that injects nitroglycerin as a liquid medicine is operated. In addition, a device capable of injecting a plurality of types of liquid medicines may be installed to be able to respond to a plurality of types of abnormalities.

Hereinbefore, a description has been given of a technology of acquiring a pulse wave form corresponding to a biological signal using, on the skin, an element capable of emitting and receiving a light ray having a particular wavelength in which absorption ability is present with respect to blood, and estimating biological information such as a blood pressure, a breathing rate, an active state of a sympathetic nerve, or the like based on the acquired pulse wave form, and use thereof. However, as an example that is not part of the present invention, the element may not be used on the skin.

As an example that is not part of the present invention, it is possible to use a combination of a light emitting device capable of widely projecting a light ray having a particular wavelength described above and a high-resolution image pick-up device capable of selectively receiving the light ray having the wavelength.

A face of a person in a particular closed space (inside a room or the like) may be identified using image processing by installing the device similarly to a monitoring camera, a change in image associated with a pulse wave of a blood flow may be treated as a biological signal, and biological information may be estimated as described above.

According to this configuration, an effort to attach a reading device to the measured person on each occasion may be saved, and thus a psychological burden of the measured person may be greatly relieved.

The above-described light emitting device and image pick-up device may be set as an inner surface 3D scanner that emits a laser ray having a particular wavelength. A person inside a particular closed space may be similarly identified using image processing and shape processing, a change in image associated with a pulse wave of a blood flow may be treated as a biological signal, and biological information may be estimated as described above.

In the above-described embodiments, a description has been given using a case, in which optics is used as a blood flow detection scheme, as an example. The description uses a principle in which an optical property of blood or a blood vessel changes with the occasion, and is highly compatible with an object of performing noninvasive measurement at all times.

However, as an example that is not part of the present invention, a scheme using a principle other than optics may be employed as the blood flow detection scheme.

For example, similar blood flow detection may be performed even when a minute pressure sensor or microphone is used around a blood vessel since a pulse wave corresponding to a change in blood flow is propagation of deformation of the blood vessel, and thus the deformation may be detected as a pressure or an oscillating wave around the blood vessel.

In addition, as an example that is not part of the present invention, accuracy increases when cardiac electricity is used in order to detect a heart rate as biological information. For this reason, a metal electrode may be provided as a sensor, and a biological signal may be acquired as an electric signal on a living body surface.

Further, as an example that is not part of the present invention, a case in which a lot of electrolyte is contained in blood and an ionized substance moves in blood is equivalent to a case in which a minute current is generated, and it is clear that there is a correlation between blood flow volume and the amount of minute current corresponding to the blood flow volume. Therefore, it is possible to detect a magnetic field generated by a minute current when a magnetic sensor is used as a sensor, and to acquire blood flow volume based on the detected magnetic field.

Additionally, acquired biological information is transmitted through communication with a device outside the biological information reading device. An electromagnetic wave such as radio, light, or the like is suitably used for communication. The electromagnetic wave may be used as a driving power source of the biological information reading device in addition to communication. The driving power source is a wireless feeder in the case of radio, and is a solar cell in the case of light. The driving power source feeds power to the biological information reading device. However, the device may operate without a battery. Further, a secondary battery may be employed as a power source of the device, and the secondary battery may be charged.

At the time of wireless communication, an antenna of the biological information reading device may be electromagnetically coupled to the blood vessel of the living body to obtain a biological antenna. In the present invention, it is presumed that noninvasive measurement is performed at all times. Thus, direction connection to the blood vessel is not performed. In addition, a film electrode is attached on the skin to share a room with another sensor around the blood vessel (for example, of the wrist), and the electrode is subjected to capacitive coupling with the blood vessel to allow the blood vessel to function as a part of an antenna. As another method, for example, when a sensor having a shape of a band of a watch is mounted on the wrist, a coil may be formed inside the band and subjected to inductive coupling with the blood vessel inside the wrist, thereby allowing the blood vessel to function as a part of an antenna.

Note that, since the blood vessel has a lot of bifurcations in shapes of branches, a place corresponding to the same length (and a length in which a standing wave ratio falls within a range allowed as an electric circuit) as a wavelength (and an integer ratio multiple of the wavelength) corresponding to a radio frequency used for communication is present. Thus, a wavelength (= frequency) may be freely selected without considering the standing wave ratio and the like. Therefore, it is possible to employ a scheme that can be used for communication widely from a plurality of schemes.

Additionally, another sensor may be provided to accessorily operate as a biological signal acquisition unit simultaneously with identification of the blood flow. Examples of the sensor include a microphone, a pressure sensor, a muscle potential sensor, a cardiac potential sensor, an ultrasonic wave Doppler sensor, an angle sensor, an acceleration sensor, a temperature sensor, a flow sensor, a body water sensor, a body fat sensor, a sweat rate sensor, a blood component sensor, an air temperature sensor, a humidity sensor, an atmospheric pressure sensor, an illuminance sensor, a wind velocity sensor, and the like.

The pressure sensor may acquire a variation in heart beat or pulse pressure as a biological signal. The microphone may acquire cardiac sound or pulse sound as a biological signal. The muscle potential sensor or the cardiac potential sensor may acquire a biological signal such as a so-called electrocardiogram or electromyogram. The ultrasonic wave Doppler sensor may acquire blood flow volume as a biological signal. The angle sensor or the acceleration sensor may identify an active state such as exercise. The temperature sensor may acquire a body temperature as a biological signal. The flow sensor may more directly identify a respiration state. The body water sensor or the sweat rate sensor may identify water content inside the body or on a surface of the body. The body fat sensor may acquire biological information in the form of a body fat percentage. The blood component sensor may acquire a blood sugar level or blood pH as a biological signal.

The air temperature sensor, the humidity sensor, the atmospheric pressure sensor, the illuminance sensor, or the wind velocity sensor may identify a living environment of the measured person.

When a signal obtained by these types of sensors is appropriately used, it is possible to further increase accuracy in analyzing information obtained from a blood flow or a pulse wave.

### {Reference Signs List}

- 1, 1a, 1b, 1c, 1d: biological information reading device
- 2: biological information acquisition unit
- 3: projector
- 4: optical receiver
- 10: light emitting element (polarized light emitter)
- 11: polarizing plate
- 12, 12a, 12b, 12c: light receiving element (light receiver, polarized light receiver)
- 13: quarter-wave plate (polarization property change portion)
- 14: permeable membrane
- 20: pulsation detector
- 21: blood pressure estimation unit
- 22, 22a: alert issuing unit
- 23: output unit (portion of alert issuing unit)
- 24: operation unit
- 25: battery

## Claims

1. A biological information reading device (1, 1a, 1b, 1c, 1d) for reading biological information, comprising:
a biological signal acquisition unit (2) configured to acquire a biological signal from a living body;
an operation unit (24) configured to perform an operation of estimating biological information based on an acquired biological signal corresponding to the biological signal acquired by the biological signal acquisition unit (2); and
a biological information output unit (23) configured to output estimated biological information corresponding to the biological information estimated by the operation unit to an outside of the biological information reading device,
which is **characterized in that**
the biological signal acquisition unit includes a stacked light emitting and receiving element in which a light receiving element (12, 12c), a polarizing plate (11, 11a) an organic light emitting diode (10) implementing a linearly polarized light emitting function, and a quarter-wave plate (13) are stacked such that linearly polarized light emitted from the organic light emitting diode (10) is changed by the quarter-wave plate (13) to light circularly polarized and that reflected light from the living body is changed by the quarter-wave plate (13) to light linearly polarized in a different direction from the light emitted from the organic light emitting diode (10) to pass through the organic light emitting diode (10) and the polarizing plate (11, 11a) to the light receiving element (12, 12c), and
a light transmissive adhesive layer (14) is provided on a surface of the quarter-wave plate (13) of the biological signal acquisition unit coming into contact with a skin of the living body, wherein the quarter-wave plate (13) and the light transmissive adhesive layer (14) have refractive indices matching with the refractive index of skin of the living body.

2. The biological information reading device (1a) according to claim 1, wherein the polarizing plate (11, 11a) and the light receiving element (12, 12c) are replaced by a polarized light receiver (12a) having a polarization property in sensitivity thereof different from a polarization property of the outward light emitted by the organic light emitting diode (10).

3. A biological information reading device (1, 1a, 1b, 1c, 1d) according to claim 1 or 2, wherein the light receiving element (12, 12a, 12c) includes an organic CMOS element.

4. The biological information reading device (1, 1a, 1b, 1c, 1d) according to any one of preceeding claims, comprising:
an estimated biological information time series storage unit configured to successively store the estimated biological information subjected to the operation of the operation unit over time.

5. The biological information reading device (1, 1a, 1b, 1c, 1d) according to claim 4, comprising:
a determination unit configured to determine a state of the living body based on the estimated biological information; and
a determination information holding unit configured to hold information necessary for the determination unit to perform a determination.

6. The biological information reading device (1, 1a, 1b, 1c, 1d) according to claim 5, comprising:
a message output unit (23) configured to output a message according to a determination result of the determination unit.

7. The biological information reading device (1, 1a, 1b, 1c, 1d) according to claim 6, wherein the message is represented by any one of or a combination of a plurality of a sense of sight, an auditory sense, an olfactory sense, a sense of taste, and a sense of touch.

## Patentansprüche

1. Vorrichtung zum Abtasten biologischer Information (1, 1a, 1b, 1c, 1d), um biologische Information abzutasten, aufweisend:
eine biologische Signalerfassungseinheit (2), welche dafür ausgelegt ist, ein biologisches Signal von einem lebenden Körper zu erfassen;
eine Betriebseinheit (24), welche dafür ausgelegt ist, um einen Vorgang des Abschätzens der biologischen Information auf der Grundlage eines erfassten, biologischen Signals durchzuführen, welches dem von der biologischen Signalerfassungseinheit (2) erfassten biologischen Signal entspricht; und
eine biologische Informationsausgabeeinheit (23), welche dafür ausgelegt ist, um die abgeschätzte, biologische Information, welche der von der Betriebseinheit abgeschätzten, biologischen Information entspricht, nach Außen von der Vorrichtung zum Abtasten biologischer Information auszugeben,
welche **dadurch gekennzeichnet ist, dass**
die biologische Signalerfassungseinheit ein geschichtetes Licht ausstrahlendes und empfangendes Element (12, 12c) aufweist, in welchem eine Polarisationsplatte (11, 11a), eine organische Leuchtdiode (10), welche eine linear polarisierte Licht ausstrahlende Funktion implementiert, und eine Viertelwellenplatte (13) derartig geschichtet sind, dass linear polarisiertes Licht, welches von der organischen Leuchtdiode (10) ausgestrahlt wird, durch die Viertelwellenplatte (13) in zirkular polarisiertes Licht verändert wird, und dass von dem lebenden Körper reflektiertes Licht durch die Viertelwellenplatte (13) in linear polarisiertes Licht in einer von dem Licht, welches von der organischen Leuchtdiode (10) ausgestrahlt wird, verschiedenen Richtung verändert wird, um durch die organische Leuchtdiode (10) und zu dem Licht empfangenden Element (12, 12c) zu gelangen, und
eine lichtdurchlässige Klebstoffschicht (14) auf einer Oberfläche der Viertelwellenplatte (13) der biologischen Signalerfassungseinheit vorgesehen ist, welche mit einer Haut des lebenden Körpers in Kontakt kommt, wobei die Viertelwellenplatte (13) und die lichtdurchlässige Klebstoffschicht (14) Brechungsindices aufweist, welche mit dem Brechungsindex der Haut des lebenden Körpers übereinstimmen.

2. Vorrichtung zum Abtasten biologischer Information (1a) nach Anspruch 1, wobei die Polarisationsplatte (11, 11a) und das Licht empfangende Element (12, 12c) durch einen polarisierten Lichtempfänger (12a) ersetzt sind, welcher eine Polarisationseigenschaft bei der Empfindlichkeit davon aufweist, welche von einer Polarisationseigenschaft des durch die organische Leuchtdiode (10) nach außen ausgestrahlten Lichts verschieden ist.

3. Vorrichtung zum Abtasten biologischer Information (1, 1a, 1b, 1c, 1d) nach Anspruch 1 oder 2, wobei das Licht empfangende Element (12, 12a, 12c) ein organisches CMOS-Element aufweist.

4. Vorrichtung zum Abtasten biologischer Information (1, 1a, 1b, 1c, 1d) nach einem der vorhergehenden Ansprüche, aufweisend:
eine Speichereinheit für abgeschätzte, biologische Informationszeitfolgen, welche dafür ausgelegt ist, um die abgeschätzte, biologische Information fortlaufend zu speichern, welche dem Vorgang der Betriebseinheit im Laufe der Zeit unterworfen ist.

5. Vorrichtung zum Abtasten biologischer Information (1, 1a, 1b, 1c, 1d) nach Anspruch 4, aufweisend:
eine Bestimmungseinheit, welche dafür ausgelegt ist, um einen Zustand des lebenden Körpers auf der Grundlage der abgeschätzten, biologischen Information zu bestimmen; und
eine Bestimmungsinformationshalteeinheit, welche dafür ausgelegt ist, um Information zu halten, welche für die Bestimmungseinheit notwendig ist, um eine Bestimmung durchzuführen.

6. Vorrichtung zum Abtasten biologischer Information (1, 1a, 1b, 1c, 1d) nach Anspruch 5, aufweisend:
eine Meldungsausgabeeinheit (23), welche dafür ausgelegt ist, um eine Meldung gemäß einem Bestimmungsergebnis der Bestimmungseinheit auszugeben.

7. Vorrichtung zum Abtasten biologischer Information (1, 1a, 1b, 1c, 1d) nach Anspruch 6, wobei die Meldung durch irgendeines oder eine Kombination von einer Vielzahl von einem Sehvermögen, einem Gehörsinn, einem Geruchssinn, einem Geschmackssinn und einem Tastsinn dargestellt wird.

## Revendications

1. Un dispositif de lecture d'informations biologique (1, 1a, 1b, 1c, 1d) pour la lecture d'informations biologiques, comprenant :
une unité d'acquisition de signal biologique (2) configurée de sorte à acquérir un signal biologique d'un organisme vivant ;
une unité de commande (24) configurée de sorte à effectuer une opération consistant à estimer des informations biologiques sur la base d'un signal biologique acquis correspondant au signal biologique acquis par l'unité d'acquisition de signal biologique (2) ; et
une unité de sortie d'informations biologiques (23) configurée de sorte à envoyer des informations biologiques estimées par l'unité de commande à l'extérieur du dispositif de lecture d'informations biologique,
**caractérisé en ce que**
l'unité d'acquisition de signal biologique comprend un élément de réception et d'émission de lumière empilé dans lequel sont empilés un élément récepteur de lumière (12, 12c), une lame polarisante (11, 11a), une diode électroluminescente organique (10) remplissant une fonction d'émission de lumière polarisée linéaire, et une lame quart d'onde (13) de sorte que la lumière polarisée émise de manière linéaire émise par la diode électroluminescente organique (10) soit transformée par la lame quart d'onde (13) en lumière polarisée de manière circulaire et que la lumière réfléchie par l'organisme vivant soit transformée par la lame quart d'onde (13) en lumière polarisée de manière linéaire dans une direction différente de la lumière émise par la diode électroluminescente organique (10) afin de passer à travers la diode électroluminescente organique (10) et la lame polarisante (11, 11a) jusqu'à l'élément récepteur de lumière (12, 12c), et
une couche adhésive transmettant la lumière (14) est prévue sur une surface de la lame quart d'onde (13) de l'unité d'acquisition de signal biologique entrant en contact avec la peau de l'organisme vivant, étant précisé que la lame quart d'onde (13) et la couche adhésive transmettant la lumière (14) présentent des indices de réfraction correspondant aux indices de réfraction de la peau de l'organisme vivant.

2. Le dispositif de lecture d'informations biologique (1a) selon la Revendication 1, étant précisé que la lame polarisante (11, 11a) et l'élément récepteur de lumière (12, 12c) sont remplacés par un récepteur de lumière polarisée (12a) présentant une propriété de polarisation dont la sensibilité est différente de la propriété de polarisation de la lumière sortante émise par la diode électroluminescente organique (10).

3. Un dispositif de lecture d'informations biologiques (1, 1a, 1b, 1c, 1d) selon la Revendication 1 ou 2, étant précisé que l'élément récepteur de lumière (12, 12a, 12c) comprend un élément CMOS organique.

4. Un dispositif de lecture d'informations biologique (1, 1a, 1b, 1c, 1d) selon l'une quelconque des Revendications précédentes, comprenant :
une unité de stockage temporel d'information biologique estimée configurée de sorte à stocker successivement l'information biologique estimée soumise à l'opération de l'unité de commande dans le temps.

5. Le dispositif de lecture d'informations biologique (1, 1a, 1b, 1c, 1d) selon la Revendication 4, comprenant :
une unité de détermination configurée de sorte à déterminer un état de l'organisme vivant sur la base des informations biologiques estimées ; et
une unité de conservation des informations de détermination configurée de sorte à conserver les informations nécessaires pour que l'unité de détermination puisse effectuer une détermination.

6. Le dispositif de lecture d'informations biologique (1, 1a, 1b, 1c, 1d) selon la Revendication 5, comprenant :
une unité d'envoi de message (23) configurée de sorte à envoyer un message en fonction d'un résultat de détermination obtenu par l'unité de détermination.

7. Le dispositif de lecture d'informations biologique (1, 1a, 1b, 1c, 1d) selon la Revendication 6, étant précisé que le message est représenté par l'un quelconque ou la combinaison de plusieurs sens parmi le sens de la vue, le sens de l'audition, le sens du goût et le sens du toucher.
